(19) Europäisches Patentamt
European Patent Office
Office européen des brevets

(11) Numéro de publication : 0 455 528 B1

# (12) FASCICULE DE BREVET EUROPEEN

(45) Date de publication du fascicule du brevet : **14.07.93 Bulletin 93/28**

(51) Int. Cl.$^5$ : **A61K 7/00**, A61K 9/127, A61K 9/10

(21) Numéro de dépôt : **91400925.3**

(22) Date de dépôt : **05.04.91**

## (54) Composition cosmétique ou dermopharmaceutique contenant des vésicules formées par un mélange phospholipides/glycolipides.

Le dossier contient des informations techniques présentées postérieurement au dépôt de la demande et ne figurant pas dans le présent fascicule.

(30) Priorité : **30.04.90 FR 9005470**

(43) Date de publication de la demande : **06.11.91 Bulletin 91/45**

(45) Mention de la délivrance du brevet : **14.07.93 Bulletin 93/28**

(84) Etats contractants désignés : **AT BE CH DE DK ES FR GB GR IT LI NL SE**

(56) Documents cités :
**EP-A- 0 043 327**
**EP-A- 0 306 971**
**EP-A- 0 365 868**
**DE-A- 3 413 541**

(56) Documents cités :
**DE-A- 3 433 609**
**FR-A- 2 614 787**
**PATENT ABSTRACTS OF JAPAN vol. 12, no. 373 (C-534)(3220) 6 octobre 1988, & JP-A-63 126820 (SHISEIDO CO LTD) 30 mai 1988,**

(73) Titulaire : **L'OREAL**
**14, Rue Royale**
**F-75008 Paris (FR)**

(72) Inventeur : **Handjani, Rose-Marie**
**17 Bis, rue Campagne Première**
**F-75014 Paris (FR)**
Inventeur : **Ribier, Alain**
**2, boulevard Jourdan**
**F-75014 Paris (FR)**
Inventeur : **Colarow, Ladislas**
**Eden-Roc**
**CH-121073 Savigny (CH)**

(74) Mandataire : **Peuscet, Jacques et al**
**Cabinet Peuscet 68, rue d'Hauteville**
**F-75010 Paris (FR)**

EP 0 455 528 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition cosmétique ou dermopharmaceutique contenant des vésicules lipidiques dispersées dans une phase aqueuse continue.

On sait que l'on a déjà proposé, depuis longtemps, l'utilisation dans des compositions cosmétiques de vésicules lipidiques dispersées dans une phase aqueuse continue, lesdites vésicules ayant une structure en feuillets constituée de deux ou plusieurs couches lipidiques séparées les unes des autres par des couches de phase aqueuse. Ces vésicules peuvent ainsi servir à encapsuler dans les compartiments aqueux compris entre les couches lipidiques, des substances actives hydrosolubles (voir notamment FR-2 315 991). On a déjà proposé par ailleurs, l'utilisation de ces vésicules pour maintenir la stabilité d'une dispersion, dans une phase aqueuse, d'une phase liquide non-miscible à l'eau (voir notamment FR-2 490 504). On a, en conséquence, proposé des compositions cosmétiques comportant en dispersion aqueuse, d'une part, des vésicules lipidiques et, d'autre part, des gouttelettes d'une phase non-miscible à l'eau, lesdites gouttelettes étant notamment susceptibles de renfermer des substances cosmétiquement actives. Dans l'état de la technique, on a proposé comme lipides amphiphiles susceptibles de constituer les vésicules de ces compositions, soit des lipides ioniques, soit des lipides non-ioniques, les vésicules des deux types pouvant être mélangées à l'intérieur d'une même composition.

Jusqu'à présent, et selon les buts particuliers poursuivis, on a proposé l'utilisation de certains lipides ioniques ou non-ioniques choisis pour leurs performances particulières et, pour chaque type d'utilisation, on a proposé d'ajouter à ces lipides amphiphiles, des additifs tels que par exemple le cholestérol ou le dicétylphosphate, dont la nature et la quantité étaient choisies en fonction des propriétés particulières que l'on désirait pour les vésicules dans l'application considérée.

DE-A 3 433 609 décrit un perfectionnement à un procédé de fabrication d'un lait ou d'une crème de régime dans lequel on prépare des liposomes par homogénéisation ou sonification de lait ou de crème sans extraction préalable des lipides complexes."

Les vésicules que l'on utilise dans le domaine cosmétique et dermopharmaceutique doivent essentiellement posséder quatre qualités ci-après énumérées :

- en premier lieu, les vésicules doivent avoir une capacité d'encapsulation aussi importante que possible, notamment, vis-à-vis des solutés hydrosolubles ;
- en second lieu, les vésicules doivent avoir une taille stable en fonction du temps et de la température de conservation pour que les compositions restent constantes au cours du stockage ;
- en troisième lieu, les vésicules doivent être stables dans leur composition chimique en fonction du temps et de la température de conservation, notamment en ce qui concerne la teneur en peroxydes pour que, là encore, les compositions restent stables en cours de stockage ;
- en quatrième lieu, les vésicules doivent être bien tolérées par la peau et, en particulier, leurs lipides constitutifs doivent avoir une cytotoxicité nulle ou très faible vis-à-vis des cellules de l'épiderme et du derme.

Jusqu'à ce jour, les vésicules réalisées avec les lipides d'origine naturelle et additifs connus ne présentaient pas simultanément les quatre qualités ci-dessus énumérées et l'on agissait sur le choix des lipides et des additifs et sur leurs proportions, pour trouver un équilibre aussi satisfaisant que possible compte-tenu du but recherché dans chaque cas particulier pour l'application cosmétique. Néanmoins, il était évidemment extrêmement souhaitable de pouvoir disposer d'un matériau lipidique d'origine naturelle susceptible, d'une part, de permettre la formation de vésicules et, d'autre part, de conférer, par sa nature même, aux dites vésicules l'ensemble des quatre qualités susmentionnées.

L'invention a pour but de proposer une composition cosmétique ou dermopharmaceutique qui contient des vésicules présentant simultanément les quatre qualités susmentionnées, le niveau atteint pour chacune de ces qualités étant du même ordre de grandeur que le meilleur niveau atteint pour la qualité en cause par l'un quelconque des lipides amphiphiles d'origine naturelle connus pour former des vésicules. Selon l'invention, on propose d'utiliser, comme lipides amphiphiles vésiculaires d'origine naturelle, les lipides complexes du type de ceux gui se trouvent dans les membranes des globules gras d'un lait de mammifère. On a constaté que, de façon surprenante, l'utilisation de ces lipides complexes dans une proportion appropriée permettait d'obtenir simultanément les meilleurs niveaux pour les quatre qualités précédemment mentionnées.

On sait qu'un lait de mammifère contient, en milieu aqueux, outre du lactose et des protéines, une quantité importante de lipides. Ces lipides sont constitués de deux catégories : d'une part, en prépondérance, les lipides neutres, constitués notamment de triglycérides et de stérols, et, d'autre part, des lipides complexes, en quantité moindre, ces lipides complexes formant des éléments constitutifs des membranes des globules gras du lait. Ces lipides membranaires sont eux-mêmes constitués de deux catégories : d'une part, les phospholipides et, d'autre part, les glycolipides.

Les phospholipides sont des dérivés phosphorylés d'un alcool, à savoir le glycérol (glycérophospholipide)

ou la sphingosine (sphingophospholipide); dans le premier cas, les deux fonctions alcool non phosphorylées du glycérol sont estérifiées par des acides gras ; dans le deuxième cas, la fonction amine de la sphingosine est liée à un acide gras par une liaison amide. Dans tous les cas, les phospholipides comportent donc dans leurs molécules une ou deux chaînes grasses. Dans les phospholipides du lait des mammifères, ces chaînes grasses sont linéaires et pour la plupart en $C_{16}$-$C_{22}$.

Les glycolipides ont une structure qui dérive de celle de la sphingosine en liant un acide gras sur la fonction amine de la sphingosine par une liaison amide et en fixant sur le groupe hydroxyle primaire de la sphingosine un ou plusieurs oses. Dans cette catégorie, si l'on fixe au moins un résidu osidique, soit le glucose, soit le galactose, on obtient la sous-classe des cérébrosides ; si l'on fixe, outre les résidus osidiques, un reste hexosamine et un reste acide sialique, on obtient des gangliosides. Les gangliosides sont donc une sous-classe des glycolipides.

On a constaté (et cela constitue la base de l'invention), que si l'on effectue une extraction des lipides complexes, qui se trouvent dans les membranes des globules gras d'un lait de mammifère et si l'on utilise ces lipides complexes pour constituer des vésicules, on obtient simultanément, au meilleur niveau, les quatre qualités requises pour les vésicules. Dans une telle extraction faite à partir du lait, les proportions relatives des phospholipides et des glycolipides, dans le produit extrait, sont quelque peu fonction de l'extraction et de la matière première utilisée. On a constaté, en outre, selon l'invention, que, dès lors que les lipides complexes utilisés pour constituer les vésicules comportent de 50 à 75 % en poids de phospholipides et de 50 à 25 % en poids de glycolipides, dont au plus 5 % en poids de gangliosides, quelle(s) que soi(en)t la (ou les) matière(s) première(s) d'origine, on obtient le résultat de qualité susmentionné. Les vésicules correspondantes sont, pour la suite, appelées "lactovésicules", indépendamment de la (ou des) matière(s) première(s) effectivement utilisée(s).

On a déjà décrit, dans la demande de brevet européen 89-118 183.6, la séparation hors d'un mélange lipidique des lipides complexes contenus dans ce mélange ; on a également décrit la séparation dans ces lipides complexes, d'une part, des phospholipides, d'autre part, des glycolipides et enfin, des gangliosides. Il est donc possible à l'homme de métier de séparer ces trois fractions et de reconstituer la formulation pondérale ci-dessus indiquée pour obtenir, quelles que soient les matières premières d'origine, une formulation de lipides complexes, qui permette de fabriquer des lactovésicules pour les compositions selon l'invention. Mais il est possible également, de façon plus simple, de choisir une matière première qui permet, par la simple extraction des lipides complexes, d'obtenir un rapport pondéral satisfaisant entre les phospholipides et les glycolipides et un rapport pondéral satisfaisant entre les gangliosides et les glycolipides totaux. Selon l'invention, on propose d'utiliser, comme matière première, un lait de mammifère et, plus particulièrement, des dérivés du lait tels que le babeurre, le lactosérum ou le lait écrémé. Des résultats particulièrement intéressants ont été obtenus en choisissant, comme matière première, un babeurre issu d'un lait de vache.

On a constaté que, lorsque les lipides complexes utilisés comportaient des chaînes grasses polyinsaturées, les vésicules obtenues avec de tels lipides complexes présentaient des avantages supplémentaires très intéressants pour l'application cosmétique. On sait, en effet, que certains tissus humains, tels que le cerveau et la cornée, sont particulièrement riches en acides gras polyinsaturés; or, on a constaté que la diminution avec le vieillissement de la teneur en acides gras polyinsaturés pouvait être corrélée avec divers troubles fonctionnels (dommages ischemiques cérébraux, cataracte). Lorsque des lipides complexes contenant des acides gras polyinsaturés sont utilisés, selon l'invention, pour constituer des lactovésicules mises en oeuvre dans des compositions cosmétiques ou dermopharmaceutiques, on obtient des effets bénéfiques liés au rôle de ces acides gras polyinsaturés, notamment dans la régulation de l'homéostasie épidermique, dans la fluidité et la perméabilité cellulaire, dans la régulation et l'inhibition de l'inflammation cutanée.

Dans ce domaine, on a constaté que l'on pouvait obtenir des résultats particulièrement intéressants lorsque l'on met en oeuvre, pour constituer des lactovésicules, des lipides complexes comportant un pourcentage de chaînes grasses polyinsaturées compris entre 15 et 25 % et, de préférence, voisin de 19 % de l'ensemble des chaînes grasses des lipides complexes. Cette caractéristique est atteinte naturellement pour les lipides complexes des membranes des globules gras d'un lait de mammifère et, notamment, pour ceux qui sont extraits d'un babeurre de lait de vache.

On a constaté, par ailleurs, que la présence de gangliosides, parmi les glycolipides des lipides complexes utilisés pour la constitution des vésicules dans les compositions selon l'invention favorise l'obtention simultanée des qualités des vésicules desdites compositions. En effet, les gangliosides permettent de conférer aux vésicules une charge électronégative suffisante (potentiel zeta de -50 millivolts ± 14 millivolts) pour assurer leur stabilité par inhibition du phénomène de floculation ; mais cette charge est masquée par la présence des résidus osidiques portés par la molécule à l'extrémité du squelette de la sphingosine, ce qui limite les interactions avec d'autres molécules chargées telles que les protéines. Cet effet des gangliosides a été mis en évidence par la prolongation du temps de demi-vie des liposomes dans le flux sanguin (voir notamment la communication

de J. Senior et G. Gregoriadis, faite au Colloque "Liposomes Research Days" à l'Université de Floride (Gainefville - Etats-Unis d'Amérique) (28.02 - 02.03.1990)).

La présente invention a, en conséquence, pour objet une composition cosmétique ou dermopharmaceutique consistant en une dispersion aqueuse de vésicules délimitées par des parois formées de couches moléculaires lipidiques organisées, caractérisée par le fait qu'au moins 90 % en poids des couches constitutives des parois d'au moins certaines des vésicules de la dispersion, dites "lactovésicules", sont constitués par des lipides complexes comportant de 50 à 75 % en poids de phospholipides et de 50 à 25 % en poids de glycolipides, dont au plus 5 % en poids sont constitués par des gangliosides, les pourcentages ci-dessus définis étant donnés par rapport au poids total des lipides complexes, l'éventuel complément étant constitué de produits liposolubles.

L'extraction des lipides complexes à partir d'une matière première naturelle, notamment dérivée du lait, peut être effectuée par tout procédé connu, par exemple celui décrit dans la demande de brevet EP-A-365868 ; on peut également utiliser une extraction à l'acétone ou au gaz carbonique supercritique, suivie d'une étape d'hydrolyse enzymatique.

Dans un mode préféré de réalisations, les gangliosides constituent de 1 à 5 % en poids par rapport au poids total des lipides complexes des lactovésicules ; les lipides complexes des lactovésicules de la composition selon l'invention, sont ceux dont le mélange se trouve dans les membranes des globules gras d'un lait de mammifère ; ces lipides complexes peuvent être extraits d'un babeurre, d'un lactosérum ou d'un lait écrémé. On préfère que les lipides complexes des lactovésicules comporte un poids de phospholipides sensiblement égal au double du poids des glycolipides ; dans l'ensemble des lipides complexes des lactovésicules, les gangliosides représentent avantageusement environ 3 % du poids.

On a constaté qu'il était avantageux que les chaînes grasses des lipides complexes des lactovésicules comportent de 15 à 25 % et, de préférence, environ 19 % de chaînes polyinsaturées.

On a constaté que l'on obtenait d'excellents résultats en, utilisant des lactovésicules, dont les lipides complexes sont obtenus directement par extraction à partir d'un babeurre de lait de vache.

Dans les compositions selon l'invention, le diamètre moyen des lactovésicules est avantageusement compris entre 10 et 1.000 nm. La quantité de lactovésicules présentes à l'intérieur d'une composition selon l'invention peut varier dans de larges limites, mais généralement la composition contient de 0,1 à 20 % en poids de lipides complexes constituant les parois des lactovésicules par rapport au poids total de la composition.

La préparation des lactovésicules de la composition selon l'invention s'effectue en mettant en oeuvre l'un quelconque des procédés connus pour obtenir une dispersion aqueuse de vésicules à partir de lipides amphiphiles ; on peut notamment utiliser le procédé revendiqué dans FR-2 315 991 ou les modes opératoires décrits dans FR-2 221 122.

La composition selon l'invention peut également contenir au moins une catégorie de vésicules, autres que les lactovésicules, constituées de lipides ioniques ou de lipides non ioniques ou encore d'un mélange de lipides ionique(s) et non-ionique(s), le total des lipides vésiculaires de l'ensemble de la composition étant au plus égal à 25 % du poids total de la composition. De préférence, ces vésicules additionnelles ont des diamètres moyens compris entre 10 et 1.000 nm. On peut avantageusement prévoir, dans ce cas, de préparer séparément les vésicules additionnelles et de mélanger la dispersion aqueuse correspondante à celle gui contient les lactovésicules pour obtenir la composition selon l'invention.

On peut également prévoir que la composition selon l'invention contienne de 1 à 40 % en poids d'au moins une phase dispersée non-miscible à l'eau, la proportion pondérale de tous les lipides vésiculaires par rapport à la (ou aux) phase(s) dispersée(s) étant comprise entre 0,2/1 et 1/1 ; on préfère que les gouttelettes de phase(s) dispersée(s) aient un diamètre moyen compris entre 100 et 10.000 nm.

Pour constituer la phase non-miscible à l'eau, qui est dispersée sous forme de gouttelettes comme ci-dessus indiqué, on peut avantageusement choisir au moins un composé pris dans le groupe formé par les hydrocarbures, les carbures halogénés, les polysiloxanes, les polydiméthylsiloxanes, les esters d'acides organiques ou minéraux, les éthers et les polyéthers. Parmi les hydrocarbures, on peut citer l'héxadécane et l'huile de paraffine ; parmi les carbures halogénés, on peut citer la perflurotributylamine et le perfluoro-décahydronaphtalène, les fluorocarbures, les fluoroesters, les fluoroéthers et les fluorosilicones. Lorsque la phase dispersée non-miscible à l'eau est un ester d'acide gras et de polyol, elle peut avantageusement être choisie parmi les triglycérides liquides et les esters d'acides gras et d'alcool ramifié de formule : R-COO-R', formule dans laquelle R représente le reste d'un acide gras comportant de 8 à 20 atomes de carbone et R' représente une chaîne hydrocarbonée ramifiée contenant de 3 à 20 atomes de carbone, ou dans laquelle R représente le reste d'un acide gras insaturé en $C_{20}$-$C_{22}$ et R', le reste d'un alcool gras. Parmi les esters d'acide gras et de polyol, on peut mentionner les huiles de tournesol, de maïs, de soja, de courge, de pépins de raisin, de macadamia, de noisette, de poissons et le tricaprocaprylate de glycérol. Parmi les esters d'acide gras supérieur et d'alcool ramifié, on peut mentionner l'huile de purcellin. Parmi les esters d'acides gras insaturés et d'alcool gras, on

peut mentionner l'huile de jojoba.

lorsque la composition selon l'invention contient des gouttelettes d'une phase dispersée non-miscible à l'eau, la préparation de ladite composition s'effectue, de façon connue, en deux temps : dans un premier temps, on prépare la dispersion aqueuse de vésicules, qu'il s'agisse d'une dispersion contenant uniquement, des lactovésicules ou d'une dispersion contenant également des vésicules additionnelles ; dans un second temps, on ajoute à cette dispersion, la phase non-miscible à l'eau et on la disperse en gouttelettes par agitation

La composition selon l'invention peut contenir au moins une substance cosmétiquement ou dermopharmaceutiquement active. Selon une première variante, la substance active est stockée dans les vésicules de la composition, les parois vésiculaires protégeant ladite substance active. La substance active peut être hydrosoluble auquel cas, elle peut se trouver en solution dans la phase aqueuse qui est encapsulée à l'intérieur des vésicules de la composition. Cependant, la substance active peut également être liposoluble : elle est alors stockée dans les parois lipidiques des lactovésicules. Dans le cas où la composition comporte des vésicules additionnelles, autres que les lactovésicules, la substance active pourra être stockée dans les mêmes conditions dans ces vésicules additionnelles; mais si la composition comprend une phase dispersée non-miscible à l'eau, la substance active peut également être stockée dans les gouttelettes de la phase dispersée. Lorsque la substance active est hydrosoluble, elle peut également se trouver dans la phase aqueuse continue de la composition selon l'invention.

L'incorporation de ces substances actives dans des dispersions aqueuses contenant des vésicules a déjà été décrite dans l'état de la technique, par exemple dans FR-2 485 921.

Parmi les substances actives liposolubles, on peut notamment citer des filtres-solaires, tels que le paradiméthylaminobenzoate de 2-éthylhexyle, des substances destinées à améliorer l'état des peaux sèches ou séniles, en particulier, des insaponifiables, tels que des insaponifiables de soja, de riz, de karité et d'avocat, des tocophérols, le nicotinate de tocophérol, les vitamines E, F ou A et ses esters, l'acide rétinoïque, des antioxydants, des acides gras essentiels, l'acide glycyrrhétinique, des kératolytiques, des caroténoïdes, le β carotène, le γ orizanol, les céramides et le glycyrrhétinate de stéaryle.

Parmi les substances actives hydrosolubles, on peut citer des humectants, tels que la glycérine, le sorbitol, le pentaérythritol, l'inositol, l'acide pyrrolidone carboxylique et ses sels, des agents de brunissage artificiel, tels que la dihydroxyacétone, l'érythrulose, le glycéraldéhyde, les γ-dialdéhydes, tels que l'aldéhyde tartrique, des agents de coloration de la peau, des filtres solaires, des antiperspirants, des déodorants, des astringents, des produits rafraîchissants, toniques, cicatrisants, kératolytiques, dépilatoires, des extraits de tissus animaux ou végétaux, du liquide amniotique, des polysaccharides, des agents anti-séborréhiques, des agents oxydants, tels que l'eau oxygénée, ou réducteurs, tels que l'acide thioglycolique et ses sels. Dans le domaine dermopharmaceutique, on peut aussi mentionner, comme substances actives hydrosolubles, les vitamines, les hormones, les enzymes, les vaccins, les anti-inflammatoires, les antibiotiques, les bactéricides, les dérivés du sélénium, les hydroxyacides tels que l'acide glycolique , les esters nicotiniques, le glycérol, les capteurs de radicaux libres, les dépigmentants, et les amincissants.

La composition selon l'invention peut contenir, dans sa phase continue, au moins un additif pris dans le groupe formé par les gélifiants, les agents d'alcalinisation ou d'acidification, les conservateurs, les colorants, les opacifiants et les parfums. Lorsque la composition comprend une phase non-miscible à l'eau dispersée sous forme de gouttelettes, l'additif est, de préférence, ajouté à la dispersion en même temps que la phase non-miscible à l'eau. Le gélifiant peut être introduit à une concentration variant entre 0,1 et 2 % en poids par rapport au poids total de la composition. Parmi les gélifiants utilisables, on peut citer les dérivés de cellulose, tels que l'hydroxyéthylcellulose ; des polymères synthétiques ; des dérivés d'algues, tels que le satiagum, ou encore, des gommes naturelles, telles que l'adragante. On utilise avantageusement un mélange d'acides carboxyvinyliques disponible dans le commerce sous le nom de "CARBOPOL 940" vendu par la société "GOODRICH".

Le pH des compositions selon l'invention est, de préférence, compris entre 4 et 9.

Pour mieux faire comprendre l'invention, on va en décrire maintenant plusieurs modes de réalisation définis dans les exemples ci-après.

<u>EXEMPLE 1</u> : <u>Préparation des lipides complexes constituant les parois des lactovésicules</u>

On utilise comme matière première du babeurre doux provenant de lait de vache. De façon connue, on décaséine et on délactose le babeurre par diafiltration ; puis on le sèche par pulvérisation. On obtient une poudre de babeurre ayant la composition suivante :

| COMPOSANTS | % |
|---|---|
| Humidité (2 heures d'étuve à 102°C) | 3,2 |
| Solides totaux | 96,8 |
| dont : | |
| Lipides totaux (méthode Mojonnier) | 61,24 |
| Protéines (N x 6,38) | 32,43 |
| Lactose (déterminée enzymatiquement) | 1,24 |
| Cendres | 1,87 |

On homogénéise 100 g de cette poudre en deux fois, chaque fois avec 500 ml d'un mélange hexane/méthanol (rapport volumique 94/6) pendant une minute à 50°C dans un moulin colloïdal. A chaque fois, on essore par centrifugation l'homogénéisat pendant deux minutes à 1500 g. On concentre ensuite le surnageant jusqu'à siccité sous vide à 65°C. On obtient ainsi les lipides totaux, qui restent fluides à cause de leur haut pourcentage d'huile de beurre.

Dans une deuxième étape, on effectue la séparation des lipides complexes à partir des lipides totaux. Pour ce faire, on refroidit les lipides totaux obtenus à 40°C, on les homogénéise avec 110 ml d'acétone dans un moulin colloïdal, on les laisse atteindre la température ambiante (23°C) puis on les centrifuge pendant une minute à 2000 g. On élimine le surnageant limpide, qui contient la plupart des lipides neutres et des composés odorants ; le résidu solide de la centrifugation contient les lipides complexes. On redissout ce résidu solide dans l'hexane puis on le concentre par évaporation sous vide. On obtient ainsi une matière insoluble dans l'acétone ; on suspend cette matière dans trois fois son volume d'hexane, puis on sépare les lipides complexes (environ 80 %) des lipides neutres (environ 20%) par chromatographie liquide d'absorption en utilisant 11 g de gel de silice prétraitée. La silice utilisée a une granulométrie correspondant à la fraction retenue entre les tamis de 27 et 91 mailles/cm ; elle est commercialisée, entre autres, par la société "MERCK" ; le prétraitement consiste à chauffer la silice à 165°C pendant 16 heures. Au cours de la chromatographie, les lipides neutres se sorbent sur la silice et les lipides complexes, qui forment des micelles inverses, ne se sorbent pas et sont donc élués.

Les lipides complexes ainsi obtenus (17,5 g) sont homogénéisés dans dix fois leur volume d'eau distillée et l'émulsion est ensuite lyophilisée.

Dans les lipides complexes ainsi obtenus, on détermine la présence de 66 % de phospholipides et de 33 % de glycolipides, parmi lesquels 3 % de gangliosides, tous ces pourcentages étant exprimés par rapport au poids total des lipides complexes.

On a déterminé le pourcentage de chaînes grasses insaturées par rapport à l'ensemble des chaînes grasses pour chaque longueur de chaîne dans les phospholipides et les glycolipides qui constituent les lipides complexes ainsi obtenus. Le résultat de cette détermination est fourni ci-après :

| Nature du lipide | | Pourcentage de chaînes grasses insaturées par rapport à l'ensemble des chaînes grasses de la catégorie | | | | | |
|---|---|---|---|---|---|---|---|
| | | $C_{18}$ | $C_{20}$ | $C_{20}$ | $C_{22}$ | $C_{22}$ | $C_{22}$ |
| | | i = 3 | i = 3 | i = 4 | i = 4 | i = 5 | i = 6 |
| Origine : Babeurre | Phospho-lipides | 1,5 | 0,6 | 0,8 | 5,7 | 0,6 | 0,1 |
| | Glyco-lipides totaux | 1,3 | 0,9 | 1,2 | 4,8 | 1,2 | 0,2 |

i = nombre d'insaturations dans la chaîne insaturée

On voit que, dans les lipides complexes obtenus, 19 % environ des chaînes grasses des phospholipides et glycolipides sont des chaînes grasses polyinsaturées, les insaturations étant en ω3 et en ω6 (ω représentant la position de la première double liaison à partir de l'extrémité méthyle).

EXEMPLE 2 : Tests des propriétés des lactovésicules

A partir des lipides complexes obtenus à l'exemple 1, on prépare des lactovésicules en utilisant le procédé de préparation revendiqué dans FR-2 315 991. On procède de la même manière avec des lipides amphiphiles de comparaison.

Dans le tableau I donné ci-après, on compare, d'une part, les capacités d'encapsulation des différents lipides testés et, d'autre part, le maintien de l'encapsulation dans les vésicules au cours du temps. Les vésicules des dispersions aqueuses testées ont un diamètre moyen d'environ 1.500 nm. Le taux d'encapsulation est repéré par le taux de gonflement exprimé en microlitre/mg : il indique le nombre de microlitres que l'on peut encapsuler dans les vésicules en utilisant 1 mg de lipides constitutifs. On voit que les lipides complexes de l'exemple 1 ont, parmi les différents lipides amphiphiles testés, la meilleure capacité d'encapsulation.

L'étude de la capacité des vésicules à maintenir leur encapsulation au cours du temps, se déduit des valeurs fournies pour les taux de fuite à une heure, un jour, huit jours et trente jours. Le taux de fuite a été déterminé en encapsulant dans les vésicules une solution aqueuse de glucose à 54 g/l et en mesurant la quantité de glucose, qui passe dans la phase aqueuse continue après filtration des vésicules sur colonne de gel chromatographique "SEPHADEX G 50". On constate que, là encore, la qualité des résultats correspondant aux lipides complexes de l'exemple 1 est comparable à la meilleure du tableau (correspondant aux extraits de lipides totaux de cerveau de boeuf).

On a, par ailleurs, étudié la stabilité dimensionnelle au cours du temps des vésicules de petites tailles obtenues à l'ultradisperseur de type "Virtis". Dans le tableau II, la colonne de gauche indique la taille initiale des vésicules testées. On a mesuré les tailles des vésicules au bout d'un mois et deux mois de stockage ; le stockage a été effectué à une température de stockage θ égale à 4, 20, 37 ou 45°C. On voit que, pour les lipides complexes de l'exemple 1, les résultats correspondent à une parfaite stabilité dimensionnelle et se trouvent donc au niveau de qualité des meilleurs résultats obtenus avec les autres lipides testés. Les lactovésicules des compositions selon l'invention ont donc une taille parfaitement stable au cours du temps.

## TABLEAU I

| LIPIDE VESICULAIRE | | | COMPOSITION DU LIPIDE % EN POIDS | | | | GONFLEMENT EN µl/mg | TAUX DE FUITES EN % | | | |
|---|---|---|---|---|---|---|---|---|---|---|---|
| ORIGINE | NOM COMMERCIAL | FOURNISSEUR | PL | GL | (1) GG | CH | | à 1 H | à 1 J | à 8 J | à 30 J |
| SOJA | EPIKURON 200 | LUCAS MEYER | >97 | 0 | 0 | 0 | 0,9 | 52 | 45 | 100 | - |
| | 170 | | >96 | 0 | 0 | 0 | 1,0 | 69 | 91 | 100 | - |
| | 145 | | 85 | 0 | 0 | 0 | 0,3 | 89 | 100 | - | - |
| | 100 | | 65 | 0 | 0 | 0 | 0,6 | 60 | 100 | - | - |
| OEUF | OVOTHIN 200 | LUCAS MEYER | >92 | 0 | 0 | 0 | 0 | 100 | - | - | - |
| | 160 | | >85 | 0 | 0 | 0 | 1,7 | 60 | 65 | 100 | - |
| BABEURRE | LIPIDES TOTAUX | NESTEC | 18 | 9 | <1 | - | 0,3 | 65 | 100 | - | - |
| | LIPIDES COMPLEXES DE L'EXEMPLE 1 | | 66 | 33 | 3 | 0 | 7,4 | 10 | 21 | 43 | 73 |
| • GLYCOLIPIDES DE CERVEAU DE BOEUF | | NIKKO | 9 | 76 | - | 15 | 2,2 | 22 | 23 | 30 | 48 |
| • EXTRAITS DE LIPIDES TOTAUX DE CERVEAU DE BOEUF (RHC poudre) | | PENTAPHARM | 44 | 35 | - | 20 | 6,4 | 21 | 36 | 70 | 83 |
| • CHOLECITHINE DE MOELLE DE BOEUF | | GIVAUDAN | 39 | 20 | - | 12 | 1,1 | 26 | 21 | 32 | 85 |

PL : Phospholipides GL : Glycolipides GG : Gangliosides CH : Cholestérol

• La capacité d'encapsulation de cette matière première est inférieure à celle des lipides complexes de babeurre, malgré la présence dans la première citée, de cholestérol, lipide bien connu pour optimiser cette propriété.

(1) le % de gangliosides est compris dans le % des glycolipides

## TABLEAU II

| LIPIDE VESICULAIRE | | Temps | T = o | Diamètre moyen des vésicules (en $10^3$ nm) T = 1 mois | | | | T = 2 mois | | | |
|---|---|---|---|---|---|---|---|---|---|---|---|
| | | θ en °C | | 4 | 20 | 37 | 45 | 4 | 20 | 37 | 45 |
| Origine | Nom commercial | Fournisseur | | | | | | | | | |
| SOJA | EPIKURON 200 | LUCAS MEYER | 0,44 | F | F | F | F | F | F | F | F |
| | " 170 | | 0,14 | 0,16 | 0,16 | 0,15 | 0,21 | 0,14 | 0,14 | 0,23 | 0,43 |
| | " 145 | | 0,15 | 0,15 | 0,15 | 0,20* | 0,20* | 0,15 | 0,18* | 0,18* | 0,17* |
| | " 100 | | 0,13 | 0,13 | 0,13 | 0,14 | 0,14 | 0,13 | 0,13 | C | C |
| OEUF | OVOTHIN 200 | LUCAS MEYER | 0,31 | F | F | F | F | F | F | F | F |
| | " 160 | | 0,17 | 0,17 | 0,18 | 0,19 | 0,21 | 0,18 | 0,18 | 0,20 | 0,39 |
| BABEURRE | LIPIDES COMPLEXES DE L'EXEMPLE 1 | X | 0,25 | 0,25 | 0,25 | 0,24 | 0,23 | 0,24 | 0,24 | 0,23 | 0,22 |
| GLYCOLIPIDES DE CERVEAU DE BOEUF | | NIKKO | 0,32 | 0,39 | 0,41 | 0,44 | 0,42 | 0,45 | 0,45 | 0,44 | 0,44 |
| EXTRAITS DE LIPIDES TOTAUX DE CERVEAU DE BOEUF (RHC poudre) | | PENTAPHARM | 0,26 | 0,25 | 0,24 | 0,24 | 0,24 | 0,26 | 0,25 | 0,25 | 0,25 |
| CHOLECITHINE DE MOELLE DE BOEUF | | GIVAUDAN | 0,18 | 0,21 | 0,20 | 0,21 | 0,22 | 0,2 | 0,2 | 0,2 | 0,22 |

* Nombreux globules gras non lamellaires démontrant une modification chimique

F = Floculation, fusion

C = Cassé en deux phases (aqueuse et grasse)

Dans le tableau III, on a consigné les résultats relatifs à la stabilité chimique des vésicules en fonction du temps et de la température de conservation. Pour étudier la stabilité chimique, on a mesuré spécifiquement la teneur en peroxydes des compositions testées, au temps initial et après un mois puis deux mois de conservation. On sait que l'auto-oxydation des acides gras polyinsaturés est accompagnée d'une augmentation de l'absorbance pour une longueur d'onde de 233/234 nm. L'augmentation est proportionnelle à la quantité de peroxydes formée (voir J. A. O. C. S., Volume 63, n° 7, page 883 (Juillet 1986)). On a donc mesuré avec un spectrophotomètre l'absorbance à cette longueur d'onde; L'absorbance A est exprimée en pourcents par la relation: $A\ \% = 100 - \frac{I}{Io} 100$, où I et Io représentent respectivement. les flux transmis et incident ; elle est ici repérée en milliunités de densité optique DO (DO = log A). Le tableau III présente la valeur des absorbances en milliunités pour la longueur d'onde considérée. Les dispersions de vésicules soumises à cet essai ont été obtenues en prenant des vésicules lyophilisées et en les dispersant dans le méthanol à raison de $10^{-4}$ g/ml. Pour interpréter ce tableau, il convient pour un lipide vésiculaire donné de prendre en considération l'évolution de l'absorbance entre le temps O et le temps contrôlé et non pas la valeur absolue de cette absorbance. Lorsqu' il existe une grosse évolution au cours du temps, la composition doit être considérée comme instable ; l'instabilité a été repérée dans le tableau III en soulignant les résultats qui correspondent à une instabilité.

On constate que la stabilité chimique des lipides complexes de l'exemple 1 au cours du temps est du même niveau de qualité que les meilleurs résultats obtenus pour les lipides vésiculaires de comparaison.

TABLEAU III

| LIPIDE VESICULAIRE | | T | Absorbance à 233 nm en milliunités | | | | | | | | |
|---|---|---|---|---|---|---|---|---|---|---|---|
| | | | T = 0 | T = 1 mois | | | | T = 2 mois | | | |
| | | θ °C | | 4 | 20 | 37 | 45 | 4 | 20 | 37 | 45 |
| Origine | Nom commercial | Fournisseur | | | | | | | | | |
| SOJA | EPIKURON 200 | LUCAS MEYER | 104 | 96 | 117 | 172 | 125 | 135 | 118 | 168 | 182 |
| | " 170 | | 60 | 93 | 97 | 101 | 92 | 106 | 116 | 113 | 83 |
| | " 145 | | 87 | 126 | 76 | 72 | 72 | 124 | 140 | 140 | 137 |
| | " 100 | | | 108 | 135 | 175 | 156 | 337 | 575 | 773 | 917 |
| OEUF | OVOTHIN 200 | LUCAS MEYER | 80 | 68 | 162 | 94 | 140 | 189 | 219 | 268 | 265 |
| | " 160 | | 69 | 68 | 64 | 56 | 73 | 174 | 180 | 243 | 234 |
| BABEURRE | LIPIDES COMPLEXES DE L'EXEMPLE 1 | | 117 | 121 | 123 | 128 | 131 | 112 | 127 | 131 | 143 |
| GLYCOLIPIDES DE CERVEAU DE BOEUF | | NIKKO | 52 | 46 | 60 | 88 | 102 | 50 | 67 | 105 | 112 |
| EXTRAITS DE LIPIDES TOTAUX DE CERVEAU DE BOEUF (RHC poudre) | | PENTAPHARM | 94 | 104 | 204 | 219 | 223 | 149 | 232 | 234 | 207 |
| CHOLECITHINE DE MOELLE DE BOEUF | | GIVAUDAN | 97 | 104 | 102 | 113 | 142 | 101 | 109 | 112 | 140 |

Enfin, pour étudier la tolérance vis-à-vis de la peau et la cytotoxicité vis-à-vis des cellules de l'épiderme et du derme, on a effectué des essais sur des cultures de fibroblastes et de kératinocytes. Les résultats de ces essais sont consignés dans le tableau IV.

Les fibroblastes isolés à partir d'un fragment de peau humaine sont cultivés dans du milieu essentiel minimum (MEM) fourni par la société "GIBCO" et additionné de 10 % de sérum de veau foetal ; ils sont maintenus

dans une atmosphère humide contenant 5% de $CO_2$ à 37°C. Les cellules sont repiquées toutes les semaines et ensemencées à une densité constante ($10^6$ cellules/ 15 ml de culture). Les fibroblastes sont utilisés pour l'expérience au cinquième jour de culture.

Les kératinocytes sont également isolées à partir d'un fragment de peau humaine. Les cellules sont cultivées dans le milieu de culture MCDB 153 (faiblement calcique 0,1 mM de $CaCl_2$) fourni par la société "CLONETICS" et additionné du facteur de croissance épidermique, d'insuline, de phosphoéthanolamine, d'éthanolamine et d'extraits bovins pituitaires (70 mg de protéines par litre de milieu) ; les cellules sont maintenues dans une atmosphère humide contenant 5 % de $CO_2$ à 37°C. Les cellules sont repiquées à confluence et ensemencées à une densité constante ($10^5$ cellules pour 10 ml de milieu de culture). Les kératinocytes sont utilisées pour l'expérience à l'état de confluence.

Les lipides, qui sont testés sur ces cultures de cellules, sont mis sous forme de dispersions vésiculaires ; les essais ont été effectués à six concentrations comprises entre 1 mg/ml et $10^{-3}$ mg/ml. Pour l'étude de la cytotoxicité à 2h30, le milieu de la dispersion est le milieu MEM pour les fibroblastes et le milieu MCDB 153 pour les kératinocytes. Pour l'étude de la cytotoxicité à 48 heures, le milieu de dispersion, pour les fibro - blastes, est le milieu MEM avec 10 % de sérum de veau foetal et, pour les kératinocytes, le milieu MCDB complet (avec extraits bovins pituitaires).

Dans les conditions de culture ci-dessus indiquées, les cellules sont ensemencées après trypsination dans des puits de microplaques traitées pour culture cellulaire, à la densité de 20.000 cellules par puits. Ces cellules sont cultivées pendant 48 heures dans les conditions d'atmosphère humide ci-dessus indiquées. Dans chaque cas, l'étude est effectuée sur 5 micropuits et la toxicité est évaluée sur deux essais.

La viabilité cellulaire est quantifiée par une méthode colorimétrique basée sur le métabolisme mitochondrial cellulaire d'un sel de tétrazolium (à savoir le bromure de 3-(4,5-diméthylthiazol 2,5-diphényl) tétrazolium) (test de Mosmann). Au cours de la réaction, le substrat, qui est jaune pâle, est transformé par les cellules vivantes en un métabolite de couleur bleu foncé. L'absorption de ce métabolite est directement proportionnelle à la densité cellulaire. La lecture de la densité optique est effectuée dans les microplaques par un spectrophotomètre de type Elisa (Biotech EL 308) à 570 nanomètres ; comme déjà indiqué, la densité optique est le logarithme du rapport (flux incident/flux transmis). La toxicité des dispersions de lipides testées est appréciée en rapportant les densités optiques lues à celles d'un témoin traité dans les mêmes conditions. Les résultats sont exprimés en pourcentage de toxicité. Lorsqu'une toxicité importante existe, la dose léthale 50 (DL 50) approximative a été calculée par régression simple. La méthodologie utilisée est décrite en détail dans la publication "Toxic. in Vitro", Vol. 3, N° 2, p. 103-109, 1989.

Le tableau IV fournit les indications résumées que l'on peut tirer de cette expérimentation. On constate que, notamment vis-à-vis des kératinocytes, la cytotoxicité des lipides complexes de l'exemple 1 est nettement inférieure à celle des lécithines de soja utilisées comme comparaison.

Il résulte donc de cet ensemble de tests que, pour les quatre qualités requises pour des vésicules à usage cosmétique, les lipides complexes de l'exemple 1 sont au niveau le plus élevé que l'on trouve pour les différents lipides vésiculaires testés à titre de comparaison ; chaque lipide de comparaison possède de mauvaises performances concernant l'une au moins des qualités requises.

TABLEAU IV

| LIPIDE VESICULAIRE | % TOXICITE | | | |
|---|---|---|---|---|
| | FIBROBLASTES | | KERATINOCYTES | |
| Temps de contact | 2H30 | 48 H | 2H30 | 48 H |
| LECITHINE DE SOJA Lipoïd S75 à 75 % PC + 9 % PE | NT | NT | NT | DL50 à 8 $10^{-5}$g/ml |
| LECITHINE DE SOJA Van de Moortelle à 53 % PC | NT | NT | NT | DL50 à 2 $10^{-4}$g/ml |
| LECITHINE DE SOJA Van de Moortelle à 38 % PE | NT | NT | NT | DL50 à 5 $10^{-3}$g/ml |
| LECITHINE DE SOJA Van de Moortelle à 50 % PI | Toxicité dès $10^{-4}$g/ml | NT | Toxicité dès 5 $10^{-5}$g/ml | DL50 à 3 $10^{-4}$g/ml |
| LIPIDES COMPLEXES de l'exemple 1 | NT | NT | NT | NT |

NT = non toxique

EXEMPLE 3 : Liposérum pour le raffermissement de la peau utilisant des lactovésicules.

Dans un flacon en verre de 3OO ml, on pèse O,5 g des lipides complexes de l'exemple 1. On ajoute 6 g d'eau déminéralisée et on laisse gonfler le mélange pendant 1 heure à 4O°C. On homogénéise ensuite ce mélange à la spatule ; on ajoute 5 g de glycérine, 1 g d'hydroxyproline et 24,5 g d'une solution aqueuse obtenue par broyage de tissus placentaires animaux et commercialisés par la société "GATTEFOSSE" sous la dénomination "PHYLDERM". A la température ambiante, on soumet le tout à l'action d'un ultradisperseur du type "Virtis" jusqu'à ce que la taille moyenne des vésicules obtenues soit inférieure ou égale à 2OO nm. On effectue

le contrôle de la taille par un granulomètre à diffusion quasi-élastique de la lumière du type "COULTRONICS NANOSIZER".

On ajoute à la dispersion obtenue une dispersion de 0,1g de hyaluronate de sodium prégonflé dans 9,9 g d'eau déminéralisée.

On ajoute enfin à la dispersion obtenue la formulation suivante :

- Parfum 0,2 g
- Mélange d'acides carboxyvinyliques commercialisé sous le nom de "CARBOPOL 940" par la société "GOODRICH" 0,25 g
- Triéthanolamine 0,25 g
- Parahydroxybenzoate de méthyle 0,3 g
- Eau déminéralisée 52 g

On obtient ainsi un sérum fluide de couleur beige, que l'on conditionne dans un flacon à pompe doseuse manuelle. Ce sérum est utilisé en application topique biquotidienne à raison d'environ 2,5 mg par cm2 et par application. Après trois semaines d'utilisation, on observe un net raffermissement de la peau.

<u>EXEMPLE 4</u> : <u>Crème pour le soin des peaux irritées utilisant des lactovésicules.</u>

Dans un flacon en verre de 300 ml, on pèse 3 g des lipides complexes obtenus à l'exemple 1 ; on y ajoute 0,15 g d' α-tocophérol. On homogénéise les deux produits à l'aide d'une spatule. On ajoute 27 g d'eau déminéralisée et on laisse gonfler le mélange pendant 1 heure à 40°C.

On homogénéise à nouveau le mélange et on ajoute 3 g de glycérine, 0,5 g d'allantoïne et 20 g de solution aqueuse à 1% de lactate de monométhyltrisilanol (vendu par la société "EXSYMOL" sous la dénomination commerciale "LASILIUM"). En appliquant la même technique que dans l'exemple 3, on soumet le tout, à la température ambiante, à l'action d'un ultradisperseur de type "Virtis", jusqu'à ce que la taille moyenne des vésicules obtenues soit inférieure ou égale à 200 nm.

On ajoute à la dispersion obtenue 15 g d'huile de cassis, 0,5 g d'un filtre solaire commercialisé sous le nom de "UVINUL M 40" par la société "BASF", 0,5 g d'un filtre solaire commercialisé sous le nom de "PARSOL M C X" par la société "GIVAUDAN", et enfin 0,1 g de parfum. A température ambiante, on soumet le tout à l'action d'un ultradisperseur de type "Virtis" jusqu'à ce que la taille moyenne des globules d'huile soit inférieure à 500 nm.

On ajoute à la dispersion obtenue les produits suivants :

- Mélange d'acides carboxyvinyliques commercialisé sous le nom de "CARBOPOL 940" par la société "GOODRICH" 0,4 g
- Triéthanolamine 0,4 g
- Parahydroxybenzoate de méthyle 0,3 g
- Eau déminéralisée 29,60 g

On obtient ainsi une crème de couleur blanc cassé, d'une viscosité égale à 20 poises. On applique cette crème tous les matins pendant une période de trois semaines sur la peau d'un sujet à peau grasse à tendance acnéique à raison d'environ 3 mg par cm2 et par application. On constate que cette crème permet de diminuer l'irritation de la peau du sujet traité.

<u>EXEMPLE 5</u> : <u>Crème de jour pour le soin des peaux sèches utilisant des lactovésicules.</u>

Dans un flacon en verre de 100 ml, on pèse 1 g des lipides complexes obtenus à l'exemple 1 et on ajoute 5 g d'eau déminéralisée. On laisse gonfler ce mélange pendant 1 heure à 40°C. On homogénéise le mélange à la spatule et on ajoute 1 g d'hydroxyproline et 10 g d'une solution aqueuse à 1 % en poids de mannuronate de monométhyltrisilanol vendu par la société "EXSYMOL" sous la dénomination commerciale "ALGISIUM". A la température ambiante, on soumet le tout à l'action d'un ultradisperseur de type "Virtis" jusqu'à ce que la taille moyenne des vésicules obtenues soit inférieure à 200 nm en utilisant la technique définie à l'exemple 3. On obtient ainsi une première phase vésiculaire.

Dans un flacon en verre de 300 ml, on introduit les produits suivants :

- Lipide amphiphile non-ionique de formule :

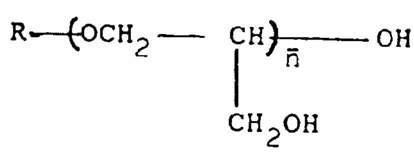

formule dans laquelle R est un radical hexadécyle et $\bar{n}$ a une valeur statistique égale à 3 O,95 g

- Cholestérol O,95 g
- Dicétylphosphate O,1O g

On réalise le mélange de ces trois produits par fusion à la température de 11O°C sous atmosphère d'azote puis on ramène la température du mélange fondu à 9O°C. On ajoute alors 3 g de glycérine dissoute dans 2O g d'eau déminéralisée. A la température de 7O°C, on soumet le tout à l'action d'un ultradisperseur de type "Virtis" jusqu'à ce que la taille moyenne des vésicules obtenues soit inférieure à 3OO nm, en appliquant la technique de contrôle décrite à l'exemple 3. On laisse revenir cette deuxième phase vésiculaire à la température ambiante.

On verse alors la première phase vésiculaire dans la seconde phase vésiculaire. On ajoute alors dans ce mélange les produits suivants :

- Huile de macadamia 14 g
- Huile de silicone volatile 1O g
- Filtre solaire commercialisé sous le nom de "UVINUL M 4O" par la société "BASF" O,5 g
- Filtre solaire commercialisé sous le nom de "PARSOL M C X" par la société "GIVAUDAN" O,5 g
- Parfum O,5 g

A une température thermostatée de 15°C, on soumet le tout à l'action d'un ultradisperseur de type "Virtis" jusqu'à ce que la taille moyenne des globules d'huile soit inférieure à 5OO nm.

On ajoute alors les produits suivants :

- Mélange d'acides carboxyvinyliques commercialisé sous le nom de "CARBOPOL 94O" par la société "GOODRICH" O,42 g
- Triéthanolamine O,42 g
- Parahydroxybenzoate de méthyle O,3 g
- Eau déminéralisée 31,36 g

On obtient ainsi une crème de couleur blanche ayant une viscosité de 3O poises.

On applique cette crème tous les matins pendant une période de trois semaines sur la peau d'un sujet à peau sèche à raison d'environ 2 mg par cm2 et par application. On constate, à la fin de la période, une réduction sensible du caractère sec de la peau.

## Revendications

### Revendications pour les Etats contractants suivants : AT, BE, CH, DE, DK, FR, GB, IT, LI, NL, SE

**1.** Composition cosmétique ou dermopharmaceutique consistant en une dispersion aqueuse de vésicules délimitées par des parois formées de couches moléculaires lipidiques organisées, caractérisées par le fait qu'au moins 90 % en poids des couches constitutives des parois d'au moins certaines des vésicules de la dispersion, dites "lactovésicules", sont constitués par des lipides complexes comportant de 50 à 75 % en poids de phospholipides et de 50 à 25 % en poids de glycolipides dont au plus 5 % en poids sont constitués par des gangliosides, les pourcentages ci-dessus définis étant donnés par rapport au poids total des lipides complexes, l'éventuel complément étant constitué de produits liposolubles.

**2.** Composition selon la revendication 1, caractérisée par le fait que les lipides complexes des lactovésicules comprennent de 1 à 5 % en poids de gangliosides..

**3.** Composition selon la revendication 2 caractérisée par le fait que les lipides complexes des lactovésicules comprennent environ 3 % en poids de gangliosides.

**4.** Composition selon l'une des revendications 1 à 3 caractérisée par le fait que les lipides complexes des lactovésicules de la composition sont ceux dont le mélange se trouve dans les membranes des globules gras d'un lait de mammifère.

**5.** Composition selon la revendication 4, caractérisée par le fait que les lipides complexes des lactovésicules de la composition sont extraits d'un babeurre, d'un lactosérum ou d'un lait écrémé.

**6.** Composition selon l'une des revendications 1 à 5, caractérisée par le fait que les lipides complexes des lactovésicules comportent un poids de phospholipides sensiblement égal au double du poids des glycolipides.

7. Composition selon l'une des revendications 1 à 6, caractérisée par le fait que les chaînes grasses des lipides complexes des lactovésicules comportent de 15 à 25 % de chaînes polyinsaturées.

8. Composition selon la revendication 7 caractérisée par le fait que les chaînes grasses des lipides complexes des lactovésicules comportent environ 19 % de chaînes polyinsaturées.

9. Composition selon les revendications 3, 6 et 8 prises simultanément, caractérisée par le fait que les lipides complexes des lactovésicules sont obtenus directement par extraction à partir d'un babeurre de lait de vache.

10. Composition selon l'une des revendications 1 à 9, caractérisée par le fait que le diamètre moyen des lactovésicules est compris entre 10 et 1.000 nm.

11. Composition selon l'une des revendications 1 à 10, caractérisée par le fait qu'elle contient de 0,1 à 20% en poids de lipides complexes constituant les parois des lactovésicules par rapport au poids total de la composition.

12. Composition selon l'une des revendications 1 à 11, caractérisée par le fait qu'elle contient au moins une catégorie de vésicules additionnelles, autres que les lactovésicules, constituées de lipides ioniques ou de lipides non-ioniques ou encore d'un mélange de lipides ionique(s) et non-ionique(s), le total des lipides vésiculaires étant au plus égal à 25 % du poids total de la composition.

13. Composition selon la revendication 12, caractérisée par le fait que les vésicules additionnelles ont un diamètre moyen compris entre 10 et 1.000 nm.

14. Composition selon l'une des revendications 1 à 13, caractérisée par le fait qu'elle contient de 1 à 40 % en poids d'au moins une phase dispersée non-miscible à l'eau, la proportion pondérale de tous les lipides vésiculaires par rapport à la (ou aux) phase(s) dispersée(s) étant comprise entre 0,2/1 et 1/1.

15. Composition selon la revendication 14 caractérisée par le fait que les gouttelettes de la phase dispersée non-miscible à l'eau ont un diamètre moyen compris entre 100 et 10.000 nm.

16. Composition selon l'une des revendications 14 ou 15, caractérisée par le fait que la phase dispersée non-miscible à l'eau est constituée par au moins un composé pris dans le groupe formé par les hydrocarbures, les carbures halogénés, les polysiloxanes, les polydiméthylsiloxanes, les esters d'acides organiques ou minéraux, les éthers et les polyéthers.

17. Composition selon la revendication 16, caractérisée par le fait que la phase dispersée non-miscible à l'eau comprend au moins un composé choisi dans le groupe formé par l'hexadécane, l'huile de paraffine, la perfluorobutylamine, le perfluorodécahydronaphtalène, les fluorocarbures, les fluoroesters, les fluoroéthers et les fluorosilicones.

18. Composition selon la revendication 16, caractérisée par le fait que la phase dispersée non-miscible à l'eau est une huile choisie dans le groupe formé par les esters d'acide gras et de polyol et les esters d'acide gras et d'alcool ramifié de formule R-COO-R', formule dans laquelle R représente le reste d'un acide gras comportant de 8 à 20 atomes de carbone et R' représente une chaîne hydrocarbonée ramifiée contenant de 3 à 20 atomes de carbone.

19. Composition selon la revendication 18, caractérisée par le fait que la phase dispersée non-miscible à l'eau comporte au moins un composé pris dans le groupe formé par les huiles de tournesol, de maïs, de soja, de courge, de pépins de raisin, de jojoba, de macadamia, de noisette, de poissons, de purcellin et le tricoprylate de glycérol.

20. Composition selon l'une des revendications 1 à 19, caractérisée par le fait qu'elle contient au moins un substance cosmétiquement ou dermopharmaceutiquement active.

21. Composition selon la revendication 20, caractérisée par le fait que la substance active est stockée dans les vésicules de la composition.

22. Composition selon la revendication 21, caractérisée par le fait que la substance active est hydrosoluble et est encapsulée à l'intérieur des vésicules de la composition.

23. Composition selon la revendication 20, caractérisée par le fait que la substance active est liposoluble.

24. Composition selon la revendication 20, caractérisée par le fait que la substance active est stockée dans les parois des vésicules de la composition.

25. Composition selon les revendications 14 et 23 prises simultanément, caractérisée par le fait que la substance active est stockée dans les gouttelettes de la phase dispersée non-miscible à l'eau.

26. Composition selon la revendication 20, caractérisée par le fait que sa phase aqueuse continue contient au moins une substance active hydrosoluble.

27. Composition selon l'une des revendications 22 ou 26, caractérisée par le fait que la substance active hydrosoluble est choisie dans le groupe formé par les humectants, les agents de brunissage artificiel, les agents de coloration de la peau, les filtres solaires hydrosolubles, les agents perspirants, les déodorants, les astringents, les produits rafraîchissants, toniques, cicatrisants, kératolytiques, dépilatoires, les extraits de tissus animaux ou végétaux, le liquide amniotique, les polysaccharides, les anti-séborrhéiques, les agents oxydants, les agents réducteurs, les vitamines, les hormones, les enzymes, les vaccins, les anti-inflammatoires, les antibiotiques, les bactéricides, les dérivés du sélénium, les hydroxyacidés, les esters nicotoniques le glycérol, les capteurs de radicaux libres, les dépigmentants et les amincissants.

28. Composition selon la revendication 23, caractérisée par le fait que la substance active liposoluble est choisie dans le groupe formé par les filtres solaires liposolubles, les substances destinées à améliorer l'état des peaux sèches ou séniles, les insaponifiables de soja, de riz, de karité et d'avocat, les tocophérols, le nicotinate de tocophérol, les vitamines E, F, ou A et ses esters, l'acide rétinoïque, les antioxydants, les acides gras essentiels, l'acide glycyrrhétinique, les kératolytiques, les caroténoïdes, le β-carotène, le γ-orizanol, les céramides et le glycyrrhétinate de stéaryle.

29. Composition selon l'une des revendications 1 à 28, caractérisée par le fait que sa phase aqueuse continue contient au moins un additif hydrosoluble pris dans le groupe formé par les gélifiants, les agents d'alcalinisation et/ou d'acidification, les conservateurs, les colorants, les opacifiants et les parfums.

30. Composition selon la revendication 29, caractérisée par le fait qu'elle contient de 0,1 à 2 % en poids, par rapport au poids total de la composition, d'au moins un gélifiant pris dans le groupe formé par les dérivés de cellulose, les polymères synthétiques, les dérivés d'algues et les gommes naturelles.

**Revendications pour les Etats constractants suivants : ES, GR**

1. Composition cosmétique consistant en une dispersion aqueuse de vésicules délimitées par des parois formées de couches moléculaires lipidiques organisées, caractérisées par le fait qu'au moins 90 % en poids des couches constitutives des parois d'au moins certaines des vésicules de la dispersion, dites "lactovésicules", sont constitués par des lipides complexes comportant de 50 à 75 % en poids de phospholipides et de 50 à 25 % en poids de glycolipides dont au plus 5 % en poids sont constitués par des gangliosides, les pourcentages ci-dessus définis étant donnés par rapport au poids total des lipides complexes, l'éventuel complément étant constitué de produits liposolubles.

2. Composition selon la revendication 1, caractérisée par le fait que les lipides complexes des lactovésicules comprennent de 1 à 5 % en poids de gangliosides..

3. Composition selon la revendication 2 caractérisée par le fait que les lipides complexes des lactovésicules comprennent environ 3 % en poids de gangliosides.

4. Composition selon l'une des revendications 1 à 3 caractérisée par le fait que les lipides complexes des lactovésicules de la composition sont ceux dont le mélange se trouve dans les membranes des globules gras d'un lait de mammifère.

5. Composition selon la revendication 4, caractérisée par le fait que les lipides complexes des lactovésicules de la composition sont extraits d'un babeurre, d'un lactosérum ou d'un lait écrémé.

6. Composition selon l'une des revendications 1 à 5, caractérisée par le fait que les lipides complexes des lactovésicules comportent un poids de phospholipides sensiblement égal au double du poids des glycolipides.

7. Composition selon l'une des revendications 1 à 6, caractérisée par le fait que les chaînes grasses des lipides complexes des lactovésicules comportent de 15 à 25 % de chaînes polyinsaturées.

8. Composition selon la revendication 7 caractérisée par le fait que les chaînes grasses des lipides complexes des lactovésicules comportent environ 19 % de chaînes polyinsaturées.

9. Composition selon les revendications 3, 6 et 8 prises simultanément, caractérisée par le fait que les lipides complexes des lactovésicules sont obtenus directement par extraction à partir d'un babeurre de lait de vache.

10. Composition selon l'une des revendications 1 à 9, caractérisée par le fait que le diamètre moyen des lactovésicules est compris entre 10 et 1.000 nm.

11. Composition selon l'une des revendications 1 à 10, caractérisée par le fait qu'elle contient de 0,1 à 20 % en poids de lipides complexes constituant les parois des lactovésicules par rapport au poids total de la composition.

12. Composition selon l'une des revendications 1 à 11, caractérisée par le fait qu'elle contient au moins une catégorie de vésicules additionnelles, autres que les lactovésicules, constituées de lipides ioniques ou de lipides non-ioniques ou encore d'un mélange de lipides ionique(s) et non-ionique(s), le total des lipides vésiculaires étant au plus égal à 25 % du poids total de la composition.

13. Composition selon la revendication 12, caractérisée par le fait que les vésicules additionnelles ont un diamètre moyen compris entre 10 et 1.000 nm.

14. Composition selon l'une des revendications 1 à 13, caractérisée par le fait qu'elle contient de 1 à 40 % en poids d'au moins une phase dispersée non-miscible à l'eau, la proportion pondérale de tous les lipides vésiculaires par rapport à la (ou aux) phase(s) dispersée(s) étant comprise entre 0,2/1 et 1/1.

15. Composition selon la revendication 14 caractérisée par le fait que les gouttelettes de la phase dispersée non-miscible à l'eau ont un diamètre moyen compris entre 100 et 10.000 nm.

16. Composition selon l'une des revendications 14 ou 15, caractérisée par le fait que la phase dispersée non-miscible à l'eau est constituée par au moins un composé pris dans le groupe formé par les hydrocarbures, les carbures halogénés, les polysiloxanes, les polydiméthylsiloxanes, les esters d'acides organiques ou minéraux, les éthers et les polyéthers.

17. Composition selon la revendication 16, caractérisée par le fait que la phase dispersée non-miscible à l'eau comprend au moins un composé choisi dans le groupe formé par l'hexadécane, l'huile de paraffine, la perfluorobutylamine, le perfluorodécahydronaphtalène, les fluorocarbures, les fluoroesters, les fluoroéthers et les fluorosilicones.

18. Composition selon la revendication 16, caractérisée par le fait que la phase dispersée non-miscible à l'eau est une huile choisie dans le groupe formé par les esters d'acide gras et de polyol et les esters d'acide gras et d'alcool ramifié de formule R-COO-R', formule dans laquelle R représente le reste d'un acide gras comportant de 8 à 20 atomes de carbone et R' représente une chaîne hydrocarbonée ramifiée contenant de 3 à 20 atomes de carbone.

19. Composition selon la revendication 18, caractérisée par le fait que la phase dispersée non-miscible à l'eau comporte au moins un composé pris dans le groupe formé par les huiles de tournesol, de maïs, de soja, de courge, de pépins de raisin, de jojoba, de macadamia, de noisette, de poissons, de purcellin et le tricoprylate de glycérol.

20. Composition selon l'une des revendications 1 à 19, caractérisée par le fait qu'elle contient au moins une substance cosmétiquement ou dermopharmaceutiquement active.

21. Composition selon la revendication 20, caractérisée par le fait que la substance active est stockée dans les vésicules de la composition.

22. Composition selon la revendication 21, caractérisée par le fait que la substance active est hydrosoluble et est encapsulée à l'intérieur des vésicules de la composition.

23. Composition selon la revendication 20, caractérisée par le fait que la substance active est liposoluble.

24. Composition selon la revendication 20, caractérisée par le fait que la substance active est stockée dans les parois des vésicules de la composition.

25. Composition selon les revendications 14 et 23 prises simultanément, caractérisée par le fait que la substance active est stockée dans les gouttelettes de la phase dispersée non-miscible à l'eau.

26. Composition selon la revendication 20, caractérisée par le fait que sa phase aqueuse continue contient au moins une substance active hydrosoluble.

27. Composition selon l'une des revendications 22 ou 26, caractérisée par le fait que la substance active hydrosoluble est choisie dans le groupe formé par les humectants, les agents de brunissage artificiel, les agents de coloration de la peau, les filtres solaires hydrosolubles, les agents perspirants, les déodorants, les astringents, les produits rafraîchissants, toniques, cicatrisants, kératolytiques, dépilatoires, les extraits de tissus animaux ou végétaux, le liquide amniotique, les polysaccharides, les anti-séborrhéiques, les agents oxydants, les agents réducteurs, les vitamines, les hormones, les enzymes, les vaccins, les anti-inflammatoires, les antibiotiques, les bactéricides, les dérivés du sélénium, les hydroxyacidés, les esters nicotoniques le glycérol, les capteurs de radicaux libres, les dépigmentants et les amincissants.

28. Composition selon la revendication 23, caractérisée par le fait que la substance active liposoluble est choisie dans le groupe formé par les filtres solaires liposolubles, les substances destinées à améliorer l'état des peaux sèches ou séniles, les insaponifiables de soja, de riz, de karité et d'avocat, les tocophérols, le nicotinate de tocophérol, les vitamines E, F, ou A et ses esters, l'acide rétinoïque, les antioxydants, les acides gras essentiels, l'acide - glycyrrhétinique, les kératolytiques, les caroténoïdes, le $\beta$-carotène, le $\gamma$-orizanol, les céramides et le glycyrrhétinate de stéaryle.

29. Composition selon l'une des revendications 1 à 28, caractérisée par le fait que sa phase aqueuse continue contient au moins un additif hydrosoluble pris dans le groupe formé par les gélifiants, les agents d'alcalinisation et/ou d'acidification, les conservateurs, les colorants, les opacifiants et les parfums.

30. Composition selon la revendication 29, caractérisée par le fait qu'elle contient de 0,1 à 2 % en poids, par rapport au poids total de la composition, d'au moins un gélifiant pris dans le groupe formé par les dérivés de cellulose, les polymères synthétiques, les dérivés d'algues et les gommes naturelles.

## Claims

### Claims for the following Contracting States : AT, BE, CH, DE, DK, FR, GB, IT, LI, NL, SE

1. Cosmetic or dermopharmaceutical composition consisting of an aqueous dispersion of vesicles delimited by walls formed of structured lipid molecular layers, characterised in that at least 90 % by weight of the constituent layers of the walls of at least some of the vesicles of the dispersion, known as "lactovesicles", consist of complex lipids containing from 50 to 75 % by weight of phospholipids and from 30 to 25 % by weight of glycolipids, not more than 5 % by weight of which consist of gangliosides, the percentages defined above being given with respect to the total weight of the complex lipids, the possible remainder consisting of fat-soluble products.

2. Composition according to Claim 1, characterised in that the complex lipids of the lactovesicles comprise from 1 to 5 % by weight of gangliosides.

3. Composition according to Claim 2, characterised in that the complex lipids of the lactovesicles comprise approximately 3 % by weight of gangliosides.

4. Composition according to one of Claims 1 to 3, characterised in that the complex lipids of the lactovesicles of the composition are those of which the mixture is found in the membranes of the fat globules of a mammalian milk.

5. Composition according to Claim 4, characterised in that the complex lipids of the lactovesicles of the composition are extracted from a buttermilk, a whey or a skimmed milk.

6. Composition according to one of Claims 1 to 5, characterised in that the complex lipids of the lactovesicles contain a weight of phospholipids approximately equal to twice the weight of the glycolipids.

7. Composition according to one of Claims 1 to 6, characterised in that the fatty chains of the complex lipids of the lactovesicles contain from 15 to 25 % of polyunsaturated chains.

8. Composition according to Claim 7, characterised in that the fatty chains of the complex lipids of the lactovesicles contain approximately 19 % of polyunsaturated chains.

9. Composition according to Claims 3, 6 and 8 taken together, characterised in that the complex lipids of the lactovesicles are obtained directly by extraction from a cow's milk buttermilk.

10. Composition according to one of Claims 1 to 9, characterised in that the mean diameter of the lactovesicles is between 10 and 1,000 nm.

11. Composition according to one of Claims 1 to 10, characterised in that it contains from 0.1 to 20 % by weight of complex lipids constituting the walls of the lactovesicles with respect to the total weight of the composition.

12. Composition according to one of Claims 1 to 11, characterised in that it contains at least one category of additional vesicles, other than the lactovesicles, consisting of ionic lipids or nonionic lipids or even of a mixture of ionic and nonionic lipids, the total of the vesicular lipids being at most equal to 25 % of the total weight of the composition.

13. Composition according to Claim 12, characterised in that the additional vesicles have a mean diameter between 10 and 1,000 nm.

14. Composition according to one of Claim 1 to 13, characterised in that it contains from 1 to 40 % by weight of at least one water-immiscible dispersed phase, the proportion by weight of all the vesicular lipids with respect to the dispersed phase(s) being between 0.2/1 and 1/1.

15. Composition according to Claim 14, characterised in that the droplets of the water-immiscible dispersed phase have a mean diameter between 100 and 10,000 nm.

16. Composition according to one of Claims 14 or 15, characterised in that the water-immiscible dispersed phase consists of at least one compound taken from the group formed by hydrocarbons, halogenated hydrocarbone, polysiloxanes, polydimethylsiloxanes, esters of organic or inorganic acids, ethers and polyethers.

17. Composition according to Claim 16, characterised in that the water-immiscible dispersed phase comprises at least one compound chosen from the group formed by hexadecane, paraffin oil, perfluorobutylamine, perfluorodecahydronaphthalene, fluorocarbons, fluoroesters, fluoroethers and fluorosilicones.

18. Composition according to Claim 16, characterised in that the water-immiscible dispersed phase is an oil chosen from the group formed by the esters of a fatty acid and a polyol and the esters of a fatty acid and a branched alcohol of formula R-COO-R', in which formula R represents the residue of a fatty acid containing from 8 to 20 carbon atoms and R' represents a branched hydrocarbon chain containing from 3 to 20 carbon atoms.

19. Composition according to Claim 18, characterised in that the water-immiscible dispersed phase contains at least one compound taken from the group formed by sun-flower oil, maize oil, soya oil, gourd oil, grapeseed oil, jojoba oil, macadamia oil, hazelnut oil, fish oil, Pur-cellin oil and glyceryl tricaprocaprylate.

**20.** Composition according to one of Claims 1 to 19, characterised in that it contains at least one cosmetically or dermopharmaceutically active substance.

**21.** Composition according to Claim 20, characterised in that the active substance is stored in the vesicles of the composition.

**22.** Composition according to Claim 21, characterised in that the active substance is water-soluble and is encapsulated inside the vesicles of the composition.

**23.** Composition according to Claim 20, characterised in that the active substance is fat-soluble.

**24.** Composition according to Clalm 20, characterised in that the active substance is stored in the walls of the vesicles of the composition.

**25.** Composition according to Claims 14 and 23 taken together, characterised in that the active substance is stored in the droplets of the water-immiscible dispersed phase.

**26.** Composition according to Claim 20, characterised in that its continuous aqueous phase contains at least one water-soluble active substance.

**27.** Composition according to one of Claims 22 or 26, characterised in that the water-soluble active substance is chosen from the group formed by moistening agents, artificial tanning agents, skin colouring agents, water-soluble sunscreen agents, antiperspirant agents, deodorants, astringents, freshening products, tonics, healing products, keratolytic products, depilatory products, extracts of animal or plant tissues, amniotic fluid, polysaccharides, anti-seborrhoeic agents, oxidising agents, reducing agents, vitamins, hormones, enzymes, vaccines, anti-inflammatories, antibiotics, bactericides, selenium derivatives, hydroxy acids, nicotinic esters of glycerol, free radical scavengers, depigmenting agents and slimming products.

**28.** Composition according to Claim 23, characterised in that the fat-soluble active substance is chosen from the group formed by the fat-soluble sunscreen agents, substances intended to improve the condition of dry or senile skins, unsaponifiable materials from soya, rice, karite and avocado, tocopherols, tocopherol nicotinate, vitamins E, F, or A and its esters, retinoic acid, antioxidising agents, essential fatty acid, glycerrhetinic acid, keratolytic agents, carotenoids, β-carotene, γ-orizanol, ceramides and stearyl glycyrrhetinate.

**29.** Composition according to one of Claims 1 to 28, characterised in that its continuous aqueous phase contains at least one water-soluble additive taken from the group formed by gelling agents, basifying and/or acidifying agents, preserving agents, dyes, opacifying agents and fragrances.

**30.** Composition according to Claim 29, characterised in that it contains from 0.1 to 20 by weight, with respect to the total weight of the composition, of at least one gelling agent taken from the group formed by cellulose derivatives, synthetic polymers, algae derivatives and natural gums.

**Claims for the following Contracting States : ES, GR**

**1.** Cosmetic composition consisting of an aqueous dispersion of vesicles delimited by walls formed of structured lipid molecular layers, characterised in that at least 90 % by weight of the constituent layers of the walls of at least some of the vesicles of the dispersion, known as "lactovesicles", consist of complex lipids containing from 50 to 75 % by weight of phospholipids and from 50 to 25 % by weight of glycolipids, not more than 5 % by weight of which consist of gangliosides, the percentages defined above being given with respect to the total weight of the complex lipids, the possible remainder consisting of fat-soluble products.

**2.** Composition according to Claim 1, characterised in that the complex lipids of the lactovesicles comprise from 1 to 5 % by weight of gangliosides.

**3.** Composition according to Claim 2, characterised in that the complex lipids of the lactovesicles comprise approximately 3 % by weight of gangliosides.

**4.** Composition according to one of Claims 1 to 3, characterised in that the complex lipids of the lactovesicles

of the composition are those of which the mixture is found in the membranes of the fat globules of a mammalian milk.

5. Composition according to Claim 4, characterised in that the complex lipids of the lactovesicles of the composition are extracted from a buttermilk, a whey or a skimmed milk.

6. Composition according to one of Claims 1 to 5, characterised in that the complex lipids of the lactovesicles contain a weight of phospholipids approximately equal to twice the weight of the glycolipids.

7. Composition according to one of Claims 1 to 6, characterised in that the fatty chains of the complex lipids of the lactovesicles contain from 15 to 25 % of polyunsaturated chains.

8. Composition according to Claim 7, characterised in that the fatty chains of the complex lipids of the lactovesicles contain approximately 19 % of polyunsaturated chains.

9. Composition according to Claims 3, 6 and 8 taken together, characterised in that the complex lipids of the lactovesicles are obtained directly by extraction from a cow's milk buttermilk.

10. Composition according to one of Claims 1 to 9, characterised in that the mean diameter of the lactovesicles is between 10 and 1,000 nm.

11. Composition according to one of Claims 1 to 10, characterised in that it contains from 0.1 to 20 % by weight of complex lipids constituting the walls of the lactovesicles with respect to the total weight of the composition.

12. Composition according to one of Claims 1 to 11, characterised in that it contains at least one category of additional vesicles, other than the lactovesicles, consisting of ionic lipids or nonionic lipids or even of a mixture of ionic and nonionic lipids, the total of the vesicular lipids being at most equal to 25 % of the total weight of the composition.

13. Composition according to Claim 12, characterised in that the additional vesicles has a mean diameter between 10 and 1,000 nm.

14. Composition according to one of Claims 1 to 13, characterised in that it contains from 1 to 40 % by weight of at least one water-immiscible dispersed phase, the proportion by weight of all the vesicular lipids with respect to the dispersed phase(s) being between 0.2/1 and 1/1.

15. Composition according to Claim 14, characterised in that the droplets of the water-immiscible dispersed phase have a mean diameter between 100 and 10,000 nm.

16. Composition according to one of Claims 14 or 15, characterised in that the water-immiscible dispersed phase consists of at least one compound taken from the group formed by hydrocarbons, halogenated hydrocarbons, polysiloxanes, polydimethylsiloxanes, esters of organic or inorganic acids, ethers and polyethers.

17. Composition according to Claim 16, characterised in that the water-immiscible dispersed phase comprises at least one compound chosen from the group formed by hexadecane, paraffin oil, perfluorobutylamine, perfluorodecahydronaphthalene, fluorocarbons, fluoroesters, fluoroethers and fluorosilicones.

18. Composition according to Claim 16, characterised in that the water-immiscible dispersed phase is an oil chosen from the group formed by the esters of a fatty acid and a polyol and the esters of a fatty acid end a branched alcohol of formula R-COO-R', in which formula R represents the residue of a fatty acid containing from 8 to 20 carbon atoms and R' represents a branched hydrocarbon chain containing from 3 to 20 carbon atoms.

19. Composition according to Claim 18, characterised in that the water-immiscible dispersed phase contains at least one compound taken from the group formed by sun-flower oil, maize oil, soya oil, gourd oil, grape-seed oil, jojoba oil, macadamia oil, hazelnut oil, fish oil, Pur-cellin oil and glyceryl tricaprocaprylate.

20. Composition according to one of Claims 1 to 19, characterised in that it contacts at least one cosmetically

or dermopharmaceutically active substance.

21. Composition according to Claim 20, characterised in that the active substance is stored in the vesicles of the composition.

22. Composition according to Claim 21, characterised in that the active substance is water-soluble and is encapsulated inside the vesicles of the composition.

23. Composition according to Claim 20, characterised in that the active substance is fat-soluble.

24. Composition according to Claim 20, characterised in that the active substance is stored in the walls of the vesicles of the composition.

25. Composition according to Claims 14 and 23 taken together, characterised in that the active substance is stored in the droplets of the water-immiscible dispersed phase.

26. Composition according to Claim 20, characterised in that its continuous aqueous phase contains at least one water-soluble active substance.

27. Composition according to one of Claims 22 or 26, characterised in that the water-soluble active substance is chosen from the group formed by moistening agents, artificial tanning agents, skin colouring agents, water-soluble sunscreen agents, antiperspirant agents, deodorants, astringents, freshening products, tonics, healing products, keratolytic products, depilatory products, extracts of animal or plant tissues, amniotic fluid, polysaccharides, anti-seborrhoeic agents, oxidising agents, reducing agents, vitamins, hormones, enzymes, vaccines, anti-inflammatories, antibiotics, bactericides, selenium derivatives, hydroxy acids, nicotinic esters of glycerol, free radical scavengers, depigmenting agents and slimming products.

28. Composition according to Claim 23, characterised in that the fat-soluble active substance is chosen from the group formed by the fat-soluble sunscreen agents, substances intended to improve the condition of dry or senile skins, unsaponifiable materials from soya, rice, karite and avocado, tocopherols, tocopherol nicotinate, vitamins E, F, or A and its esters, retinoic acid, antioxidising agents, essential fatty acids, glycerrhetinic acid, keratolytic agents, carotenoids, β-carotene, γ-orizanol, ceramides and stearyl glycyrrhetinate.

29. Composition according to one of Claims 1 to 28, characterised in that its continuous aqeous phase contains at least one water-soluble additive taken from the group formed by gelling agents, basifying and/or acidifying agents, preserving agents, dyes, opacifying agents and fragrances.

30. Composition according to Claim 29, characterised in that it contains from 0.1 to 2 % by weight, with respect to the total weight of the composition, of at least one gelling agent taken from the group formed by cellulose derivatives, synthetic polymers, algae derivatives and natural gums.

## Patentansprüche

### Patentansprüche für folgende Vertragsstaaten : AT, BE, CH, DE, DK, FR, GB, IT, LI, NL, SE

1. Kosmetische oder dermopharmazeutische Zusammensetzung, bestehend aus einer wäßrigen Dispersion von Vesikeln, welche durch Wände begrenzt sind, die aus organisierten, molekularen Lipidschichten gebildet sind, dadurch gekennzeichnet, daß wenigstens 90 Gew.-% der Schichten, welche die Wände bestimmter Vesikel bilden, die wenigstens einen Teil der Vesikel der Dispersion ausmachen und "Laktovesikel" genannt werden, aus komplexen Lipiden, die 50 bis 75 Gew.-% Phospholipide und 50 bis 25 Gew.-% Glycolipide umfassen, aufgebaut sind, von denen höchstens 5 Gew.-% Ganglioside sind und die gegebenenfalls durch fettlösliche Produkte ergänzt sind, wobei die obenerwähnten Prozentangaben auf das Gesamtgewicht der komplexen Lipide bezogen sind.

2. Zusammensetzung nach Anspruch 1, dadurch gekennzeichnet, daß die komplexen Lipide der Laktovesikel 1 bis 5 Gew.-% Ganglioside umfassen.

3. Zusammensetzung nach Anspruch 2, dadurch gekennzeichnet, daß die komplexen Lipide der

Laktovesikel etwa 3 Gew.-% Ganglioside umfassen.

4. Zusammensetzung nach einem der Ansprüche 1 bis 3, dadurch gekennzeichnet, daß es sich bei den komplexen Lipiden der Laktovesikel der Zusammensetzung um diejenigen handelt, die sich als Gemisch in den Membranen der Fettkügelchen der Milch eines Säugetieres finden.

5. Zusammensetzung nach Anspruch 4, dadurch gekennzeichnet, daß die komplexen Lipide der Laktovesikel der Zusammensetzung aus Buttermilch, Laktoserum oder Magermilch extrahiert worden sind.

6. Zusammensetzung nach einem der Ansprüche 1 bis 5, dadurch gekennzeichnet, daß die komplexen Lipide der Laktovesikel Phospholipide in einer Gewichtsmenge umfassen, die im wesentlichen gleich der doppelten Gewichtsmenge der Glycolipide ist.

7. Zusammensetzung nach einem der Ansprüche 1 bis 6, dadurch gekennzeichnet, daß die Fettketten der komplexen Lipide der Laktovesikel 15 bis 25% mehrfach ungesättigte Ketten umfassen.

8. Zusammensetzung nach Anspruch 7, dadurch gekennzeichnet, daß die Fettketten der komplexen Lipide der Laktovesikel etwa 19% mehrfach ungesättigte Ketten umfassen.

9. Zusammensetzung nach den Ansprüchen 3, 6 und 8 zusammengenommen, dadurch gekennzeichnet, daß die komplexen Lipide der Laktovesikel direkt durch Extraktion von Kuhbuttermilch erhalten worden sind.

10. Zusammensetzung nach einem der Ansprüche 1 bis 9, dadurch gekennzeichnet, daß der mittlere Durchmesser der Laktovesikel zwischen 10 und 1000 nm liegt.

11. Zusammensetzung nach einem der Ansprüche 1 bis 10, dadurch gekennzeichnet, daß sie 0,1 bis 20 Gew.-% der die Wände der Laktovesikel bildenden komplexen Lipide, bezogen auf das Gesamtgewicht der Zusammensetzung, enthält.

12. Zusammensetzung nach einem der Ansprüche 1 bis 11, dadurch gekennzeichnet, daß sie wenigstens eine von den Laktovesikeln verschiedene, zusätzliche Vesikelart enthält, die aus ionischen Lipiden oder nicht-ionischen Lipiden oder aus einer Mischung aus (einem) ionischen und nicht-ionischen Lipid(en) gebildet ist, wobei die Gesamtmenge der Vesikellipide höchstens 25 Gew-% der gesamten Zusammensetzung beträgt.

13. Zusammensetzung nach Anspruch 12, dadurch gekennzeichnet, daß die zusätzlichen Vesikel einen mittleren Durchmesser zwischen 10 und 1000 nm aufweisen.

14. Zusammensetzung nach einem der Ansprüche 1 bis 13, dadurch gekennzeichnet, daß sie 1 bis 40 Gew.-% wenigstens einer mit Wasser nicht mischbaren dispergierten Phase enthält, wobei das Gewichtsverhältnis aller Vesikellipide, bezogen auf die dispergierte(n) Phase(n), zwischen 0,2/1 und 1/1 liegt.

15. Zusammensetzung nach Anspruch 14, dadurch gekennzeichnet, daß die Tröpfchen der mit Wasser nicht mischbaren dispergierten Phase einen mittleren Durchmesser zwischen 100 und 10000 nm aufweisen.

16. Zusammensetzung nach einem der Ansprüche 14 oder 15, dadurch gekennzeichnet, daß die mit Wasser nicht mischbare dispergierte Phase aus wenigstens einer Verbindung gebildet ist, die ausgewählt ist unter Kohlenwasserstoffen, halogenierten Kohlenwasserstoffen, Polysiloxanen, Polydimethylsiloxanen, Estern organischer Säuren oder von Mineralsäuren, Äthern und Polyäthern.

17. Zusammensetzung nach Anspruch 16, dadurch gekennzeichnet, daß die mit Wasser nicht mischbare,dispergierte Phase wenigstens eine Verbindung umfaßt, die ausgewählt ist unter Hexadecan, Paraffinöl, Perfluorbutylamin, Perfluordecahydronaphthalin, Fluorkohlenstoffen, Fluorestern, Fluoräthern und Fluorsilikonen.

18. Zusammensetzung nach Anspruch 16, dadurch gekennzeichnet, daß die mit Wasser nicht mischbare, dispergierte Phase ein Öl ist, das ausgewählt ist unter Estern einer Fettsäure und eines Polyols und Estern einer Fettsäure und eines verzweigten Alkohols der Formel R-COO-R', worin R einen Fettsäurerest mit

8 bis 20 Kohlenstoffatomen und R' eine verzweigte Kohlenwasserstoffkette mit 3 bis 20 Kohlenstoffatomen bedeutet.

19. Zusammensetzung nach Anspruch 18, dadurch gekennzeichnet, daß die mit Wasser nicht mischbare dispergierte Phase wenigstens eine Verbindung umfaßt, die ausgewählt ist unter Sonnenblumen-, Mais-, Soja-, Kürbis-, Traubenkern-, Jojoba-, Macadamia-, Nuß-, Fisch-, Purcellinöl und Glycerintricaprocaprylat.

20. Zusammensetzung nach einem der Ansprüche 1 bis 19, dadurch gekennzeichnet, daß sie wenigstens eine kosmetisch oder dermopharmazeutisch wirksame Substanz enthält.

21. Zusammensetzung nach Anspruch 20, dadurch gekennzeichnet, daß die wirksame Substanz in den Vesikeln der Zusammensetzung gespeichert ist.

22. Zusammensetzung nach Anspruch 21, dadurch gekennzeichnet, daß die wirksame Substanz wasserlöslich und im Inneren der Vesikel der Zusammensetzung eingekapselt ist.

23. Zusammensetzung nach Anspruch 20, dadurch gekennzeichnet, daß die wirksame Substanz fettlöslich ist.

24. Zusammensetzung nach Anspruch 20, dadurch gekennzeichnet, daß die wirksame Substanz in den Wänden der Vesikel der Zusammensetzung gespeichert ist.

25. Zusammensetzung nach Anspruch 14 und 23 zusammengenommen, dadurch gekennzeichnet, daß die wirksame Substanz in den Tröpfchen der mit Wasser nicht mischbaren dispergierten Phase gespeichert ist.

26. Zusammensetzung nach Anspruch 20, dadurch gekennzeichnet, daß ihre kontinuierliche wäßrige Phase wenigstens eine wasserlösliche aktive Substanz enthält.

27. Zusammensetzung nach einem der Ansprüche 22 oder 26, dadurch gekennzeichnet, daß die wasserlösliche wirksame Substanz ausgewählt ist unter Feuchthaltemitteln, Mitteln zur künstlichen Bräunung, Mitteln zur Färbung der Haut, wasserlöslichen Sonnenfiltern, Perspirantien, Deodorantien, Adstringentien, erfrischenden, tonisierenden, vernarbenden, keratolytischen, depilierenden Produkten, Extrakten tierischer oder pflanzlicher Gewebe, einer amniotischen Flüssigkeit, Polysacchariden, Antiseborrhoika, Oxidationsmitteln, Reduktionsmitteln, Vitaminen, Hormonen, Enzymen, Vaccinen, anti-inflammatorischen Mitteln, Antibiotika, Bakteriziden, Selenderivaten, Hydroxysäuren, Nikotinestern von Glyzerin, Fängern von freien Radikalen, Depigmentierungsmitteln und schlankmachenden Mitteln.

28. Zusammensetzung nach Anspruch 23, dadurch gekennzeichnet, daß die fettlösliche wirksame Substanz ausgewählt ist unter fettlöslichen Sonnenfiltern, Substanzen, die zur Verbesserung des Zustandes trockener oder gealteter Haut dienen, unverseifbaren Soja-, Reis-, Karité- und Avocadobestandteilen, Tocopherolen, Tocopherolnikotinat, den Vitaminen E, F oder A und ihren Estern, Retinoesäure, Antioxidantien, essentiellen Fettsäuren, Glycyrrethinsäure, keratolytischen Mitteln, Carotinoiden, β-Carotin, γ-Orizanol, Ceramiden und Sterylglycyrrhetinat.

29. Zusammensetzung nach einem der Ansprüche 1 bis 28, dadurch gekennzeichnet, daß ihre kontinuierliche wäßrige Phase wenigstens ein wasserlösliches Additiv enthält, das ausgewählt ist unter gelbildenden Mitteln, alkalisch und/oder sauer machenden Mitteln, Konservierungsmitteln, färbenden Mitteln, opakmachenden Mitteln und Parfüms.

30. Zusammensetzung nach Anspruch 29, dadurch gekennzeichnet, daß sie 0,1 bis 2 Gew.-%, bezogen auf das Gesamtgewicht der Zusammensetzung, wenigstens eines gelbildenden Mittels enthält, das ausgewählt ist unter Zellulosederivaten, synthetischen Polymeren, Algenderivaten und natürlichen Gummis.

**Patentansprüche für folgende Vertragsstaaten : ES, GR**

1. Kosmetische Zusammensetzung, bestehend aus einer wäßrigen Dispersion von Vesikeln, welche durch Wände begrenzt sind, die aus organisierten, molekularen Lipidschichten gebildet sind, dadurch ge-kennzeichnet, daß wenigstens 90 Gew.-% der Schichten, welche die Wände bestimmter Vesikel bilden, die

wenigstens einen Teil der Vesikel der Dispersion ausmachen und "Laktovesikel" genannt werden, aus komplexen Lipiden, die 50 bis 75 Gew.-% Phospholipide und 50 bis 25 Gew.-% Glycolipide umfassen, aufgebaut sind, von denen höchstens 5 Gew.-% Ganglioside sind und die gegebenenfalls durch fettlösliche Produkte ergänzt sind, wobei die obenerwähnten Prozentangaben auf das Gesamtgewicht der komplexen Lipide bezogen sind.

2. Zusammensetzung nach Anspruch 1, dadurch gekennzeichnet, daß die komplexen Lipide der Laktovesikel 1 bis 5 Gew.-% Ganglioside umfassen.

3. Zusammensetzung nach Anspruch 2, dadurch gekennzeichnet, daß die komplexen Lipide der Laktovesikel etwa 3 Gew.-% Ganglioside umfassen.

4. Zusammensetzung nach einem der Ansprüche 1 bis 3, dadurch gekennzeichnet, daß es sich bei den komplexen Lipiden der Laktovesikel der Zusammensetzung um diejenigen handelt, die sich als Gemisch in den Membranen der Fettkügelchen der Milch eines Säugetieres finden.

5. Zusammensetzung nach Anspruch 4, dadurch gekennzeichnet, daß die komplexen Lipide der Laktovesikel der Zusammensetzung aus Buttermilch, Laktoserum oder Magermilch extrahiert worden sind.

6. Zusammensetzung nach einem der Ansprüche 1 bis 5, dadurch gekennzeichnet, daß die komplexen Lipide der Laktovesikel Phospholipide in einer Gewichtsmenge umfassen, die im wesentlichen gleich der doppelten Gewichtsmenge der Glycolipide ist.

7. Zusammensetzung nach einem der Ansprüche 1 bis 6, dadurch gekennzeichnet, daß die Fettketten der komplexen Lipide der Laktovesikel 15 bis 25% mehrfach ungesättigte Ketten umfassen.

8. Zusammensetzung nach Anspruch 7, dadurch gekennzeichnet, daß die Fettketten der komplexen Lipide der Laktovesikel etwa 19% mehrfach ungesättigte Ketten umfassen.

9. Zusammensetzung nach den Ansprüchen 3, 6 und 8 zusammengenommen, dadurch gekennzeichnet, daß die komplexen Lipide der Laktovesikel direkt durch Extraktion von Kuhbuttermilch erhalten worden sind.

10. Zusammensetzung nach einem der Ansprüche 1 bis 9, dadurch gekennzeichnet, daß der mittlere Durchmesser der Laktovesikel zwischen 10 und 1000 nm liegt.

11. Zusammensetzung nach einem der Ansprüche 1 bis 10, dadurch gekennzeichnet, daß sie 0,1 bis 20 Gew.-% der die Wände der Laktovesikel bildenden komplexen Lipide, bezogen auf das Gesamtgewicht der Zusammensetzung, enthält.

12. Zusammensetzung nach einem der Ansprüche 1 bis 11, dadurch gekennzeichnet, daß sie wenigstens eine von den Laktovesikeln verschiedene, zusätzliche Vesikelart enthält, die aus ionischen Lipiden oder nichtionischen Lipiden oder aus einer Mischung aus (einem) ionischen und nicht-ionischen Lipid(en) gebildet ist, wobei die Gesamtmenge der Vesikellipide höchstens 25 Gew-% der gesamten Zusammensetzung beträgt.

13. Zusammensetzung nach Anspruch 12, dadurch gekennzeichnet, daß die zusätzlichen Vesikel einen mittleren Durchmesser zwischen 10 und 1000 nm aufweisen.

14. Zusammensetzung nach einem der Ansprüche 1 bis 13, dadurch gekennzeichnet, daß sie 1 bis 40 Gew.-% wenigstens einer mit Wasser nicht mischbaren dispergierten Phase enthält, wobei das Gewichtsverhältnis aller Vesikellipide, bezogen auf die dispergierte(n) Phase(n), zwischen 0,2/1 und 1/1 liegt.

15. Zusammensetzung nach Anspruch 14, dadurch gekennzeichnet, daß die Tröpfchen der mit Wasser nicht mischbaren dispergierten Phase einen mittleren Durchmesser zwischen 100 und 10000 nm aufweisen.

16. Zusammensetzung nach einem der Ansprüche 14 oder 15, dadurch gekennzeichnet, daß die mit Wasser nicht mischbare dispergierte Phase aus wenigstens einer Verbindung gebildet ist, die ausgewählt ist unter Kohlenwasserstoffen, halogenierten Kohlenwasserstoffen, Polysiloxanen, Polydimethylsiloxanen, Estern organischer Säuren oder von Mineralsäuren, Äthern und Polyäthern.

**17.** Zusammensetzung nach Anspruch 16, dadurch gekennzeichnet, daß die mit Wasser nicht mischbare dispergierte Phase wenigstens eine Verbindung umfaßt, die ausgewählt ist unter Hexadecan, Paraffinöl, Perfluorbutylamin, Perfluordecahydronaphthalin, Fluorkohlenstoffen, Fluorestern, Fluoräthern und Fluorsilikonen.

**18.** Zusammensetzung nach Anspruch 16, dadurch gekennzeichnet, daß die mit Wasser nicht mischbare, dispergierte Phase ein Öl ist, das ausgewählt ist unter Estern einer Fettsäure und eines Polyols und Estern einer Fettsäure und eines verzweigten Alkohols der Formel R-COO-R', worin R einen Fettsäurerest mit 8 bis 20 Kohlenstoffatomen und R' eine verzweigte Kohlenwasserstoffkette mit 3 bis 20 Kohlenstoffatomen bedeutet.

**19.** Zusammensetzung nach Anspruch 18, dadurch gekennzeichnet, daß die mit Wasser nicht mischbare dispergierte Phase wenigstens eine Verbindung umfaßt, die ausgewählt ist unter Sonnenblumen-, Mais-, Soja-, Kürbis-, Traubenkern-, Jojoba-, Macadamia-, Nuß-, Fisch-, Purcellinöl und Glycerintricaprocaprylat.

**20.** Zusammensetzung nach einem der Ansprüche 1 bis 19, dadurch gekennzeichnet, daß sie wenigstens eine kosmetisch oder dermopharmazeutisch wirksame Substanz enthält.

**21.** Zusammensetzung nach Anspruch 20, dadurch gekennzeichnet, daß die wirksame Substanz in den Vesikeln der Zusammensetzung gespeichert ist.

**22.** Zusammensetzung nach Anspruch 21, dadurch gekennzeichnet, daß die wirksame Substanz wasserlöslich und im Inneren der Vesikel der Zusammensetzung eingekapselt ist.

**23.** Zusammensetzung nach Anspruch 20, dadurch gekennzeichnet, daß die wirksame Substanz fettlöslich ist.

**24.** Zusammensetzung nach Anspruch 20, dadurch gekennzeichnet, daß die wirksame Substanz in den Wänden der Vesikel der Zusammensetzung gespeichert ist.

**25.** Zusammensetzung nach Anspruch 14 und 23 zusammengenommen, dadurch gekennzeichnet, daß die wirksame Substanz in den Tröpfchen der mit Wasser nicht mischbaren dispergierten Phase gespeichert ist.

**26.** Zusammensetzung nach Anspruch 20, dadurch gekennzeichnet, daß ihre kontinuierliche wäßrige Phase wenigstens eine wasserlösliche aktive Substanz enthält.

**27.** Zusammensetzung nach einem der Ansprüche 22 oder 26, dadurch gekennzeichnet, daß die wasserlösliche wirksame Substanz ausgewählt ist unter Feuchthaltemitteln, Mitteln zur künstlichen Bräunung, Mitteln zur Färbung der Haut, wasserlöslichen Sonnenfiltern, Perspirantien, Deodorantien, Adstringentien, erfrischenden, tonisierenden, vernarbenden, keratolytischen, depilierenden Produkten, Extrakten tierischer oder pflanzlicher Gewebe, einer amniotischen Flüssigkeit, Polysacchariden, Antiseborrhoika, Oxidationsmitteln, Reduktionsmitteln, Vitaminen, Hormonen, Enzymen, Vaccinen, anti-inflammatorischen Mitteln, Antibiotika, Bakteriziden, Selenderivaten, Hydroxysäuren, Nikotinestern von Glyzerin, Fängern von freien Radikalen, Depigmentierungsmitteln und schlankmachenden Mitteln.

**28.** Zusammensetzung nach Anspruch 23, dadurch gekennzeichnet, daß die fettlösliche wirksame Substanz ausgewählt ist unter fettlöslichen Sonnenfiltern, Substanzen, die zur Verbesserung des Zustandes trockener oder gealteter Haut dienen, unverseifbaren Soja-, Reis-, Karité- und Avocadobestandteilen, Tocopherolen, Tocopherolnikotinat, den Vitaminen E, F oder A und ihren Estern, Retinoesäure, Antioxidantien, essentiellen Fettsäuren, Glycyrrethinsäure, keratolytischen Mitteln, Carotinoiden, $\beta$-Carotin, $\gamma$-Orizanol, Ceramiden und Sterylglycyrrhetinat.

**29.** Zusammensetzung nach einem der Ansprüche 1 bis 28, dadurch gekennzeichnet, daß ihre kontinuierliche wäßrige Phase wenigstens ein wasserlösliches Additiv enthält, das ausgewählt ist unter gelbildenden Mitteln, alkalisch und/oder sauer machenden Mitteln, Konservierungsmitteln, färbenden Mitteln, opakmachenden Mitteln und Parfüms.

**30.** Zusammensetzung nach Anspruch 29, dadurch gekennzeichnet, daß sie 0,1 bis 2 Gew.-%, bezogen auf das Gesamtgewicht der Zusammensetzung, wenigstens eines gelbildenden Mittels enthält, das ausgewählt ist unter Zellulosederivaten, synthetischen Polymeren, Algenderivaten und natürlichen Gummis.